(19) Europäisches Patentamt / European Patent Office / Office européen des brevets

(11) **EP 2 899 655 A1**

(12) # EUROPEAN PATENT APPLICATION
published in accordance with Art. 153(4) EPC

(43) Date of publication:
**29.07.2015 Bulletin 2015/31**

(21) Application number: **13838490.4**

(22) Date of filing: **20.09.2013**

(51) Int Cl.:
*G06F 19/00* (2011.01)    *G01N 15/00* (2006.01)

(86) International application number:
**PCT/JP2013/005581**

(87) International publication number:
**WO 2014/045594 (27.03.2014 Gazette 2014/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.09.2012   JP 2012208720**

(71) Applicant: **Sumitomo Heavy Industries, Ltd. Tokyo 141-6025 (JP)**

(72) Inventors:
• **OHNISHI, Yoshitaka**
**Yokosuka-Shi**
**Kanagawa 237-8555 (JP)**
• **ICHISHIMA, Daiji**
**Yokosuka-Shi**
**Kanagawa 237-8555 (JP)**

(74) Representative: **Emde, Eric**
**Wagner & Geyer**
**Gewürzmühlstrasse 5**
**80538 München (DE)**

(54) **ANALYSIS DEVICE**

(57) An analyzing device 100 analyzes a particle system defined in a virtual system by numerically calculating a governing equation that governs motion of particles in the particle system and includes: a temperature calculation unit 110 that calculates a temperature of a particle, which is one of parameters of particles in the particle system; a force calculation unit 122 that calculates a force exerted on the particle assumed to be immersed in a heat bath of the temperature calculated by the temperature calculation unit 110; and a state update unit 126 that updates a state of the particle based on the force calculated by the force calculation unit 122.

FIG.1

EP 2 899 655 A1

**Description**

[TECHNICAL FIELD]

The present invention relates to an analyzing device for analyzing a particle system.

[BACKGROUND ART]

One known method to study phenomena in material science in general based on classical mechanics or quantum mechanics and using a computer is simulation based on Molecular Dynamics Method (hereinafter, MD method), Quantum Molecular Dynamics Method, or Renormalized Molecular Dynamics (hereinafter, RMD method), which is developed from the MD method to handle a macroscale system.
Normally, the MD method and the RMD method are only capable of analyzing heat conduction by lattice vibration (phonons). Therefore, the results of analysis produced by the MD method or the RMD method in metals are often deviated from the reality because free electrons play a great role in heat conduction.
We have proposed several methods to solve the disadvantage in patent documents 1 and 2.

[patent document 1] JP2010-170309 [patent document 2] JP2012-150673
[non-patent document 1] John C. Tully, "Dynamics of gas-surface interaction: 3D generalized Langevin model applied to fcc and bcc surface", J. Chem. Phys., 1975, 73

[PROBLEM TO BE SOLVED BY THE INVENTION]

In the method described in patent document 1, parameters of temperature are assigned to particles so that a temperature field is determined by solving a heat conduction equation using Finite Volume Method (FVM). However, the resultant temperature field often does not reflect the dispersion of particle velocity so that the disclosed method has a relatively limited scope of applications.
Patent document 2 discloses a technology of converting kinetic energy of particles into random variables and delivering the energy according to the Fourier's law by using the gradient of the random variables. However, the energy may grow large depending on the gradient of the temperature, and a particle may have a negative energy. One measure to prevent this is to use small time intervals. However, this will increase the computational load.
Examples of the present invention address a need to provide an analysis technology capable of handling heat conduction phenomena in a simulation in which a particle system including a plurality of particles is used.

[MEANS TO SOLVE THE PROBLEM]

An example of the present invention relates to an analyzing device. The analyzing device that analyzes a particle system defined in a virtual system by numerically calculating a governing equation that governs motion of particles in the particle system, and includes: a temperature calculation unit that calculates a temperature of a particle, which is one of parameters of particles in the particle system; a force calculation unit that calculates a force exerted on the particle assumed to be immersed in a heat bath of the temperature calculated by the temperature calculation unit; and a state update unit that updates a state of the particle based on the force calculated by the force calculation unit.
According to the example, heat conduction in a particle system can be handled more suitably.
Note that any combination of the aforementioned components or any manifestation of the present invention realized by modifications of a method, apparatus, system, storing media, computer program, and so forth, is effective as an example of the present invention.

[ADVANTAGE OF THE PRESENT INVENTION]

According to the examples of the invention, heat conduction phenomena can be handled more accurately than the related art in a simulation using a particle system including a plurality of particles.

[BRIEF DESCRIPTION OF THE DRAWINGS]

Fig. 1 is a block diagram showing the function and configuration of an analyzing device according to a first example;
Fig. 2 is a data structure diagram showing an example of a particle data storing unit;

Fig. 3 is a flowchart showing an example of a series of steps in the analyzing device of Fig. 1;

Fig. 4 is a schematic diagram showing a particle system used in the calculation for certification according to the first example;

Fig. 5 is a graph showing results of calculation using the method according to the first example;

Fig. 6 is a graph showing results of calculation not using the method according to the first example; and

Figs. 7A-7F are schematic diagrams showing the time-dependent change of results of calculation obtained when the method according to the second example is used.

[BEST MODE FOR CARRYING OUT THE INVENTION]

In the following, same reference symbols shown in Figures indicate same or corresponding structures, parts or processes, and redundant descriptions may be omitted.

The analyzing device according to an example describes a target of analysis, using a particle system including a plurality of particles and analyzes the particle system by numerically calculating the motion equation of particles. In this process, the analyzing device determines a temperature field by solving a heat conduction equation. The analyzing device introduces, in the motion equation of particles, a force exerted on a particle that result when it is assumed that the particle is immersed in heat bath of a temperature substantially equal to the temperature as determined. More specifically, the Langevin method (see non-patent document 1), which gives a random force exerted from a particle in a heat bath and a damper resulting from viscosity, is applied. This improves physical consistency in reproducing heat conduction of a target of analysis such as metal, in which contribution from free electrons to heat conduction cannot be neglected.

(First example)

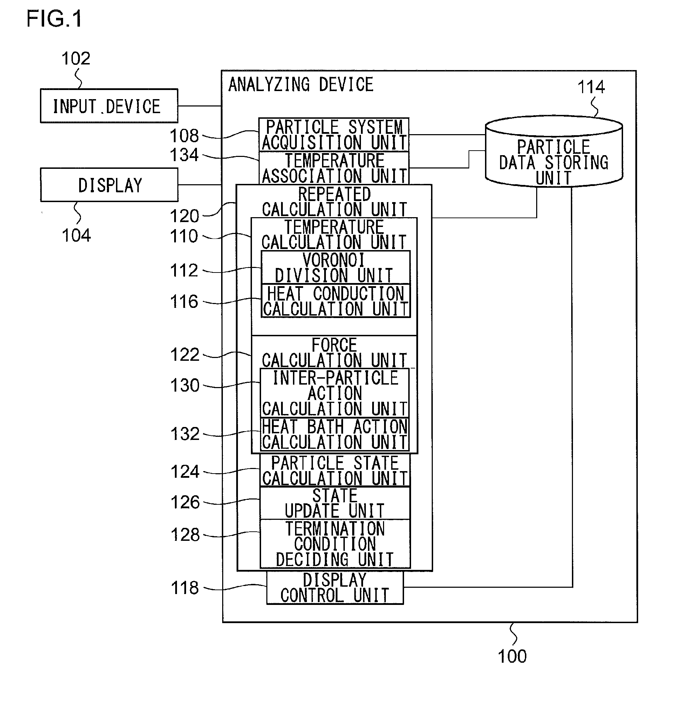

Fig. 1 is a block diagram showing the function and configuration of an analyzing device 100 according to a first example. The blocks depicted in the block diagram are implemented in hardware such as devices or mechanical components such as a CPU of a computer, and in software such as a computer program etc. Fig. 1 depicts functional blocks implemented by the cooperation of these elements. Therefore, it will be obvious to those skilled in the art having accessed this specification that the functional blocks may be implemented in a variety of manners by a combination of hardware and software.

In this example, although a case where the particle system is analyzed using the MD method is described, it is obvious to those skilled in the art having the knowledge of the specification that the technical idea of this example can be applied to a case where the particle system is analyzed using the RMD method or to a case where the particle system is analyzed using particle methods such as the Distinct Element Method (DEM), Smoothed Particle Hydrodynamics (SPH), and Moving Particle Semi-implicit (MPS).

The analyzing device 100 is connected to an input device 102 and a display 104. The input device 102 may be a keyboard or a mouse for receiving a user input related to a process performed in the analyzing device 100. The input device 102 may be configured to receive an input from a network such as the Internet or from a recording medium such as a CD, DVD, etc.

The analyzing device 100 includes a particle system acquisition unit 108, a temperature association unit 134, a repeated calculation unit 120, a display control unit 118 and a particle data storing unit 114.

The particle system acquisition unit 108 is operative to acquire data of a particle system. The particle system comprises N (N is a natural number) particles. Those N particles are defined in a one, two or three dimensional virtual space, based on input information acquired from a user through the input device 102. Particles in the particle system may be associated with molecules or atoms.

The particle system acquisition unit 108 is operative to arrange N particles in the virtual space based on the input information and to associate a velocity with each arranged particle. In other words, the particle system acquisition unit 108 assigns an initial position and an initial velocity to the particle system. The particle system acquisition unit 108 is operative to associate a particle ID identifying an arranged particle and a position of the associated particle and a velocity of the arranged particle and to register the associated information to the particle data storing unit 114.

The temperature association unit 134 associates a temperature with a particle in the particle system acquired by the particle system acquisition unit 108 based on input information acquired from a user through the input device 102. Thus, the temperature associated is one of the parameters of a particle. For example, the temperature association unit 134 prompts the user via the display 104 to input an initial value of the temperature of a particle in the particle system. The temperature association unit 134 associates the input initial value of the temperature with the particle ID and register the associated information in the particle data storing unit 114.

The following descriptions assume that all particles of the particle system are homogeneous or equivalent. The following descriptions also assume that a potential energy function is based on pair potential and has the same form regardless of particles. However, it will be obvious to a person skilled in the art who has read this specification that a technical concept according to this example can be applied to other cases.

The repeated calculation unit 120 is operative to perform numerical operation according to a governing equation that governs a motion of each particle in the particle system, the particle system being represented by data stored by the particle data storing unit 114. In particular, the repeated calculation unit 120 is operative to perform repeated operation according to an equation of motion of a discretized particle. The repeated calculation unit 120 includes a temperature calculation unit 110, a force calculation unit 122, a particle state calculation unit 124, a state update unit 126 and a termination condition deciding unit 128.

The temperature calculation unit 110 calculates the temperature of each particle in the particle system using continuum approximation. In particular, the temperature calculation unit 110 calculates the temperature of the particle based on a discretized heat conduction equation. The temperature calculation unit 110 includes a Voronoi division unit 112 and a heat conduction calculation unit 116.

The Voronoi division unit 112 performs Voronoi division of a portion of the virtual space in which the particle system is defined. In other words, the Voronoi division unit 112 creates Voronoi polyhedrons in the virtual space based on the position of the particles. First, the Voronoi division unit 112 performs three-dimensional Delauna segmentation of the portion of the virtual space with the particles at the vertices. Next, the Voronoi division unit 112 creates Voronoi polyhedron elements from tetrahedron elements obtained as a result of Delauna segmentation. This results in Voronoi division of the portion of the virtual space with the positions of the particles being kernel points.

The heat conduction calculation unit 116 calculates the temperature of each particle based on a heat conduction equation discretized by using a Voronoi polyhedron created by the Voronoi division unit 112 as a unit. The heat conduction calculation unit 116 may analyze a temperature field using the Finite Volume Method. For analysis of the temperature field, the heat conduction calculation unit 116 uses the area and volume of Voronoi polyhedrons created by the Voronoi division unit 112, and information on the temperature field at a point of time preceding the current time by a predetermined infinitesmal time interval $\Delta t$ (i.e., information on the temperature field of an immediately previous cycle in the repeated calculation). In particular, the heat conduction calculation unit 116 analyzes each of the Voronoi polyhedrons as one control volume of FVM.

The steps of deriving a discretized heat conduction equation used in the heat conduction calculation unit 116 will be shown below.

The heat conduction equation is given by the following differential equation (expression 1).

$$\rho C_V \frac{\partial T}{\partial t} = \nabla \bullet [\lambda \nabla T] + \dot{Q} \qquad (1)$$

where $\rho$ denotes density, Cv denotes specific heat, T denotes temperature, t denotes time, $\lambda$ denotes heat conductivity, and Q denotes amount of heat generated per unit volume.

Taking the volume integral of both sides of expression 1, we obtain

$$\rho \int_V C_V \frac{\partial T}{\partial t} dV = \int_V \nabla \bullet [\lambda \nabla T] dV + \int_V \dot{Q} dV \qquad (2)$$

Applying Gauss's theorem to the first term of expression 2, we obtain

$$\rho \int_V C_V \frac{\partial T}{\partial t} dV = \int_S [\lambda \nabla T] \bullet dS + \int_V \dot{Q} dV$$

Discretizing expression 3, using a Voronoi polyhedron as a unit, we obtain

$$\rho C_V \frac{T_i^{n+1} - T_i^n}{\Delta t} \Delta V_i = \sum_j \Delta S_{ij} \lambda_{ij} \frac{T_j^n - T_i^n}{r_{ij}} + \dot{Q} \Delta V_i \qquad (4)$$

where $\Delta V_i$ denotes the volume of a Voronoi polyhedron whose kernel point is located at the position of the i-th particle, $\Delta S_{ij}$ denotes the area of the Voronoi face between the i-th particle and the j-th particle, and $r_{ij}$ denotes the distance between the i-th particle and the j-th particle. $T_i^n$ denotes the temperature of the i-th particle in the n-th cycle of the repeated calculation over time (this can be said to be the temperature of the Voronoi polyhedron to which the i-th particle belongs), and $\lambda_{ij}$ denotes the heat conductivity between the i-th particle and the j-th particle. Expression 4 is a discretized heat conduction equation used in the heat conduction calculation unit 116. Expression 4 allows determining the temperature of the i-th particle in the n+1 calculation, from the volume and area of the Voronoi polyhedron, the temperature of the particles determined in the n-th cycle of calculation, and the inter-particle distance. The initial temperature associated by the temperature association unit 134 is used as $T_i^0$.

The force calculation unit 122 calculates a force exerted on a particle assumed to be immersed in a heat bath of a temperature calculated by the temperature calculation unit 110. The force calculation unit 122 includes an inter-particle action calculation unit 130 and a heat bath action calculation unit 132.

The inter-particle action calculation unit 130 is operative to refer to data of the particle system stored by the particle data storing unit 114 and to calculate a force applied to each particle in the particle system based on particle-particle distances. The inter-particle action calculation unit 130 is operative to, with regard to i-th ($1 \le i \le N$) particle in the particle system, identify particle(s) whose distance from the i-th particle is less than a predetermined cut-off distance. Hereinafter, such identified particles are called neighboring particles.

The inter-particle action calculation unit 130 is operative to calculate a force applied to the i-th particle by each neighboring particle based on the potential energy function between the neighboring particle and the i-th particle and the distance between the neighboring particle and the i-th particle. In particular, the inter-particle action calculation unit 130 is operative to calculate the force by obtaining a value of a gradient of the potential energy function at the value of the distance between the neighboring particle and the i-th particle. The inter-particle action calculation unit130 is operative to sum up the force applied to the i-th particle by a neighboring particle over all neighboring particles in order to calculate the total force applied to the i-th particle. The force calculated by the inter-particle action calculation unit 130 is a force based on the interaction between particles.

Assuming that the i-th particle is immersed in a heat bath of a substantially constant temperature at a temperature $T_i$ calculated by the temperature calculation unit 110, the following two forces are exerted on the i-th particle according to the Langevin method.

(1) Damper force (viscous force)

A damper force is a force exerted by the viscosity of the heat bath on the particle. The damping constant $\alpha$ of the viscous force is given by the equation below, using a Debye frequency $\omega_D$ and the mass m of the particle.

$$\alpha = \frac{m \pi \omega_D}{6} \qquad (5)$$

The table below lists values of damping constant $\alpha$ of typical metal substances. In this table, particles are associated with atoms.

| Substance | Debye Temperature | Debye Frequency $\omega_D$ [1/s] | Atomic weight | Mass m [kg] | Damping constant $\alpha$ [kg/s] |
|---|---|---|---|---|---|
| Al | 428 | 5.60E+13 | 26. 98 | 4.48173E-26 | 1.31E-12 |
| Ti | 420 | 5.50E+13 | 47.9 | 7.95681E-26 | 2.29E-12 |
| Cr | 630 | 8.25E+13 | 52.01 | 8.63953E-26 | 3.73E-12 |
| Fe | 470 | 6.15E+13 | 55.85 | 9.27741E-26 | 2.99E-12 |
| Ni | 450 | 5.89E+13 | 58.71 | 9.75249E-26 | 3.01E-12 |
| Cu | 343 | 4.49E+13 | 63.54 | 1.05548E-25 | 2.48E-12 |
| Zn | 327 | 4.28E+13 | 65.38 | 1.08605E-25 | 2.43E-12 |
| Mo | 450 | 5.89E+13 | 95.95 | 1.59385E-25 | 4.92E-12 |
| Ag | 225 | 2.95E+13 | 107.88 | 1.79203E-25 | 2.76E-12 |
| W | 400 | 5.24E+13 | 183.86 | 3.05415E-25 | 8.37E-12 |
| Pt | 240 | 3.14E+13 | 195.09 | 3.2407E-25 | 5.33E-12 |
| Au | 165 | 2.16E+13 | 197 | 3.27243E-25 | 3.70E-12 |

As shown in the table, the damping constant $\alpha$ is on the order of $1.0\times10^{-12}$ (kg/s) in case particles are associated with metal atoms. The Debye frequency $\omega_D$ depends on the mass of the particle. Therefore, if the mass of the particle is $\beta$ times the mass of the atom, the damping constant $\alpha$ will be $\beta^{0.5}$ times the original. For example, if particles that have the property of iron and that have a mass 100 times that of iron atoms are used, the damping constant $\alpha$ will be $2.99\times10^{-11}$ (kg/s).

(2) Random force

A random force corresponds to a force produced by collision of particles in the heat bath. The random force has a standard deviation $\sigma$ given by the expression below.

$$\sigma = \sqrt{\frac{2\alpha K_B T_i}{\Delta t}} \qquad (6)$$

where $K_B$ denotes the Boltzman constant.

The heat bath action calculation unit 132 calculates the viscous force and the random force exerted on the i-th ($1{\leq}i{\leq}N$) particle in the particle system in accordance with expressions 5 and 6. The heat bath action calculation unit 132 calculates the total force exerted on the i-th particle by adding the viscous force and the random force calculated as being exerted on the i-th particle to the force exerted on i-th particle based on the interaction between particles. The total force $F_i$ exerted on the i-th particle is given by expression 7 below.

$$\vec{F}_i = -\sum_{i\neq j}\left\{\frac{\partial \phi_{ij}(r_{ij})}{\partial r}\right\}\frac{\vec{r}_{ij}}{|r_{ij}|} - \alpha \vec{V}_i + \vec{F}_{random} \qquad (7)$$

where $\phi_{ij}$ denotes the potential energy function between the i-th particle and the j-th particle, $v_i$ denotes the velocity of the i-th particle, and $F_{random}$ denotes the random force having a standard deviation $\sigma$. The arrow over a symbol indicates a vector quantity.

The particle state calculation unit 124 refers to data for the particle system stored in the particle data storing unit 114 and calculates at least one of the position and the velocity of the particles in the particle system by applying the total force calculated by the heat bath action calculation unit 132 to the discretized motion equation of particles. In this example, the particle state calculation unit 124 calculates both the position and the velocity of the particles.

The particle state calculation unit 124 calculates the velocity of the particles using according to the discretized motion equation of particles that includes the total force calculated by the heat bath action calculation unit 132. The particle state calculation unit 124 calculates the velocity of the i-th particle in the particle system by substituting the total force calculated by the heat bath action calculation unit 132 as being exerted on the i-th particle, into the motion equation of particles discretized according to a predetermined numerical analysis method such as the leap-frog method or the Euler's method and by using a time interval $\Delta t$. In this calculation, the velocity of the particle calculated in the previous cycle of repeated calculation is used.

The particle state calculation unit 124 is operative to calculate the position of a particle based on the calculated velocity of the particle. The particle state calculation unit 124 is operative to calculate the position of the i-th particle of the particle system by applying the calculated velocity of the i-th particle to an equation of relationship between the position and the velocity of the i-th particle, the equation being discretized based on a certain numerical analysis method and the equation being discretized using the ticks of time t. This calculation uses position of the particle obtained in the previous cycle of the repeated operation.

The state update unit 126 updates the state of each particle in the particle system based on the result of calculation by the particle state calculation unit 124. The state update unit 126 is operative to update each of the position and the velocity of each particle in the particle system stored by the particle data storing unit 114 with the position and the velocity calculated by the particle state calculation unit 124.

The termination condition deciding unit 128 is operative to decide whether the repeated operation in the repeated calculation unit 120 should be terminated or not. The termination conditions with which the repeated operation should be terminated may include the condition that the number of operations in the repeated operation reaches a predetermined number, the condition that an instruction for termination is received from outside and the condition that the particle system reaches a steady state. The termination condition deciding unit 128 is operative to terminate the repeated operation in the repeated calculation unit 120 if the termination condition is met. The termination condition deciding unit 128 is operative to return the process to the temperature calculation unit 110 if the termination condition is not met. Then, the temperature calculation unit 110 is operative to again calculate the temperature with position of particles updated by the state update unit 126.

The display control unit 118 is operative to cause the display 104 to display the time evolution of the particle system or the state of the particle system at a certain time based on the position, velocity and temperature of each particle of the particle system, the particle system being represented by data stored by the particle data storing unit 114. This display may be performed in a form of still image or moving image.

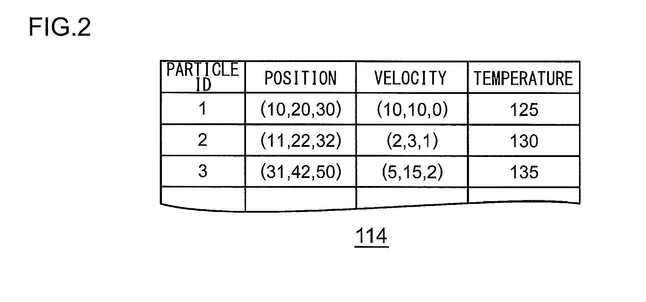

Fig. 2 is a data structure diagram showing an example of the particle data storing unit 114. The particle data storing unit 114 stores the particle ID, the position of the particle, the velocity of the particle and the temperature of the particle.

In the above-described example, an example of the storing unit is a hard disk or a memory. It should be understood by a person skilled in the art who has read this specification that it is possible to realize each unit, based on descriptions in this specification, by a CPU (not shown), a module of installed application program, a module of system program or a memory temporarily storing contents of data that has been read out from a hard disk.

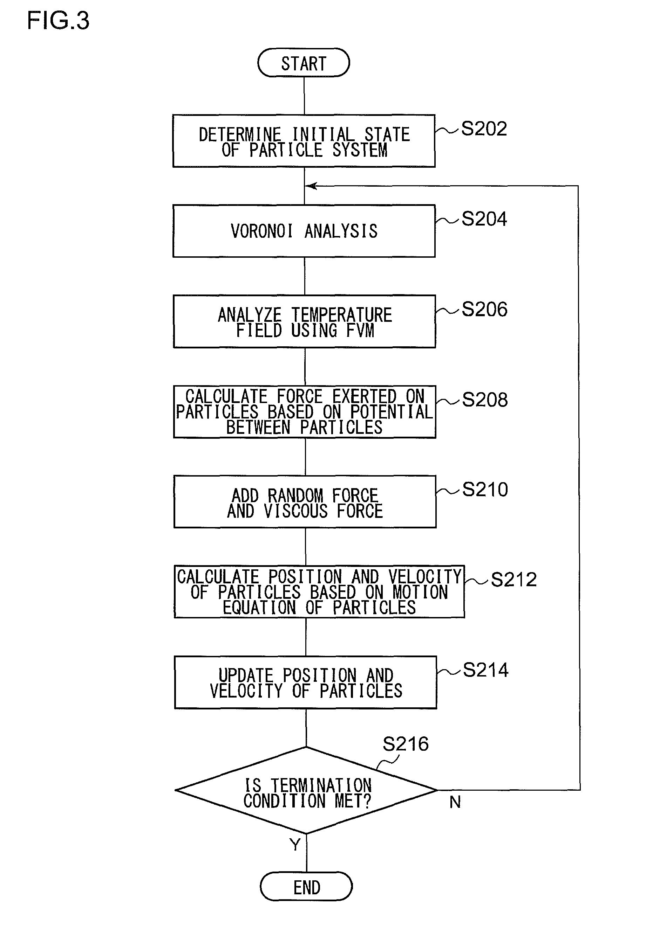

A description will now be given of the operation of the analyzing device 100 having the configuration described above. Fig. 3 is a flowchart showing an example of a series of steps in the analyzing device 100. The analyzing device 100 determines the initial state of the particle system, i.e., the initial position, the initial velocity, and the initial temperature of the particles (S202). The analyzing device 100 performs Voronoi analysis based on the position of the particles and creates Voronoi polyhedrons (S204). The analyzing device 100 uses FVM to analyze the temperature field (S206) and updates the temperature of the particles. The analyzing device 100 calculates the force exerted on each particle based on the potential energy function between particles (S208). The analyzing device 100 adds the viscous force and the random force to the force calculated in step S208 (S210). The analyzing device 100 calculates the velocity and the position of the particles according to the motion equation of particles including the force calculated in step S210 (S212). The analyzing device 100 updates the position and the velocity of the particles stored in the particle data storing unit 114 with the position and the velocity calculated (S214). The analyzing device 100 determines whether a termination condition is met (S216). If the termination condition is not met (N in S216), the process is returned to step S204. If the termination condition is met (Y in S216), the process is terminated.

The temperature calculation unit 110 calculates the temperature of each particle by continuum approximation. Therefore, the temperature of the particle calculated by the temperature calculation unit 110 may differ largely from the dispersion of particle velocity, which is the primary definition of temperature. In order to mitigate or remove such inconsistency, we have arrived at an idea of determining the temperature by the temperature calculation unit 110 and then reflecting the kinetic energy originating from the temperature in the motion of the particle. The velocity of the particle may be forced to be changed to the velocity corresponding to the temperature by, for example, temperature scaling. However, this approach places a constraint on the motion and so is non-physical in nature.

Accordingly, the analyzing device 100 according to the example is configured to correct the term of the force in the motion equation based on the temperature, by assuming that the particle is immersed in a heat bath of a temperature calculated by the temperature calculation unit 110. This can reflect the temperature calculated by the temperature calculation unit 110 in the velocity field of the particles so that the temperature field calculated by the temperature calculation unit 110 can be introduced more naturally. This can consequently provide a model with less physical inconsistency.

We conducted a calculation to certify the method according to the example. Basically, the MD method, which is incorporated in the example, is only capable of handling heat conduction by lattice vibration of particles so that contribution from free electrons is not reflected. Therefore, in case the MD method is used to analyze a metal as a target, i.e., in case material constants (e.g., Debye temperature, Debye frequency, atomic weight, and density, specific heat, heat conductivity in the heat conduction equation) are defined for particles in the particle system so that the particles simulate metal particles, the method according to the example is quite useful.

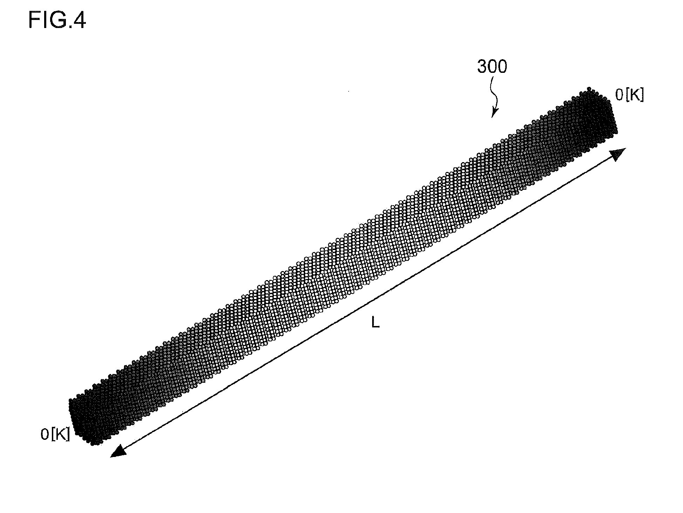

Fig. 4 is a schematic diagram showing a particle system 300 used in our calculation for certification. The particle system 300 simulates a metal bar. The temperature at the ends of the bar is fixed to 0(K). The initial temperature distribution is given by the following expression (8).

$$T(r) = 100\frac{8}{\pi^2}\sin\left\{\frac{\pi}{L}r\right\} \quad (8)$$

where L denotes the length of the bar, r denotes the distance from the end, and T(r) denotes the temperature at the distance r.

In this case, the theoretical formula of temperature distribution after the elapse of time t is given by expression 9 below.

$$T(r,t) = 100 \frac{8}{\pi^2} \exp\left(-\frac{a\pi^2}{L^2}t\right)\sin\left(\frac{\pi}{L}r\right) \qquad (9)$$

where a denotes the thermal diffusion constant, and the relationship given by the following expression 10 holds.

$$a = \frac{\lambda}{\rho C_V} \qquad (10)$$

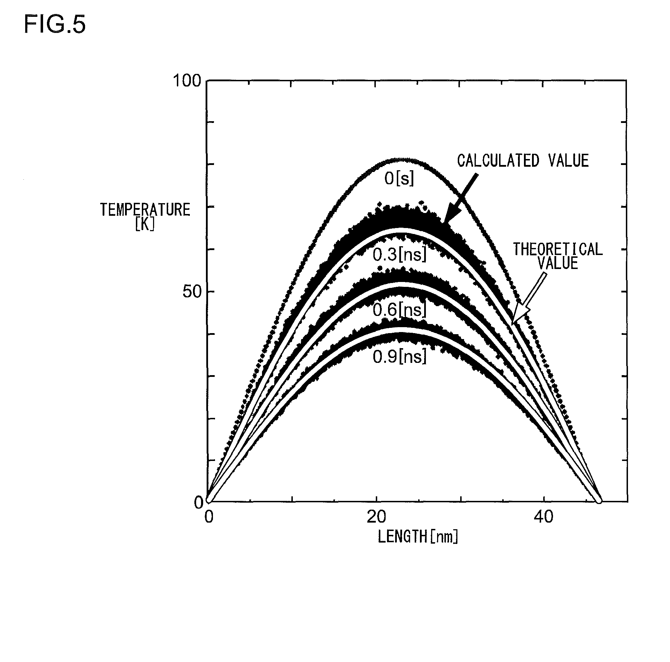

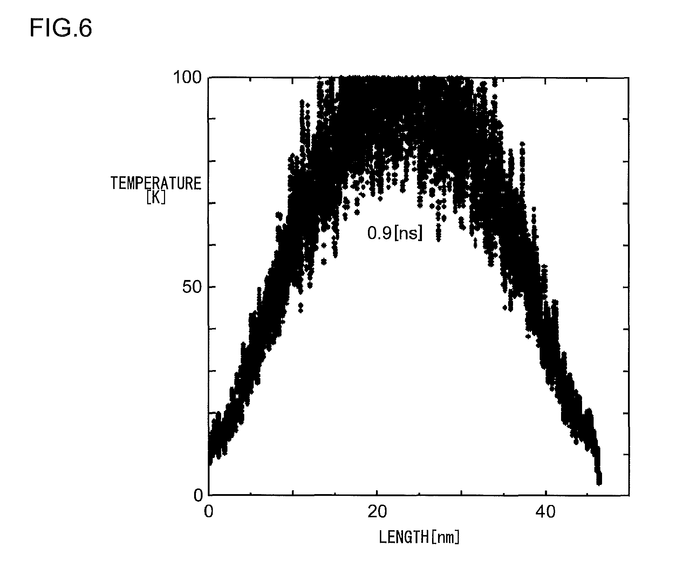

Fig. 5 is a graph showing results of calculation using the method according to the example. Fig. 6 is a graph showing results of calculation not using the method according to the example. According to the method of the example, the calculated value of temperature distribution (denoted by solid dots) agrees well with the theoretical value (denoted by the solid line) after the elapse of 0.3 (ns), 0.6 (ns), and 0.9 (ns) since the time evolution of the particle system 300 is started. This is in contrast with the case of the ordinary MD method that does not incorporate the method according to the example, where heat diffusion is extremely slower as compared with theoretical values.

(Second example)

In the second example, a description is given of a case where variation in the structure of the particle system, i.e., heat generation associated with variation in the particle arrangement, is considered. The analyzing device 100 according to the second example has the same configuration as that of Fig. 1. The following description focuses on the difference from the first example.

As in the first example, the heat conduction calculation unit 116 calculates the temperature of each particle according to a heat conduction equation discretized by using a Voronoi polyhedron created by the Voronoi division unit 112 as a unit. As in the first example, the discretized heat conduction equation used in the heat conduction calculation unit 116 is given by expression 4. In this example, heat generation associated with variation in the structure of the particle system is considered so that the amount of heat generated Q of expression 4 is given by the following expression 11.

$$\dot{Q} = \frac{\left[\sum_{i\neq j}\phi(r_{ij}) + K_i\right]^n - \left[\sum_{i\neq j}\phi(r_{ij}) + K_i\right]^{n-1}}{\Delta t \bullet \Delta V_i} + \frac{\sum_{i\neq j}F_{rij}\bullet v_{rij}}{\Delta V_i} \qquad (11)$$

where $\phi$ denotes the potential energy function between particles, $K_i$ denotes the kinetic energy of the i-th particle, n denotes the number of times of repeated calculation with time, $\Delta t$ denotes the time interval, $F_{rij}$ denotes the friction created between the i-th particle and the j-th particle, and $v_{rij}$ denotes the relative velocity between the i-th particle and the j-th particle. Expression 11 indicates that heat is generated if the total energy of the particles is increased due to deformation of the particle system. Expressions 4 and 11 determine the temperature of the i-th particle in the n+1-th calculation.

As in the first example, the analyzing device 100 according to this example is capable of reflecting the temperature calculated by the temperature calculation unit 110 in the temperature field of the particles so that the temperature field calculated by the temperature calculation unit 110 can be introduced more naturally. This can consequently provide a model with less physical inconsistency. In further accordance with the analyzing device 100 of this example, heat generation associated with variation in the structure of the particle system can be reflected in the temperature calculated by the temperature calculation unit 110 (heat conduction calculation unit 116). Accordingly, there is provided a model with less physical inconsistency in the presence of deformation in the structure of the particle system. This allows more accurate simulation of a phenomenon in which heat generation associated with deformation of a metal such as plastic forming is involved and allows prediction of temperature increase during work.

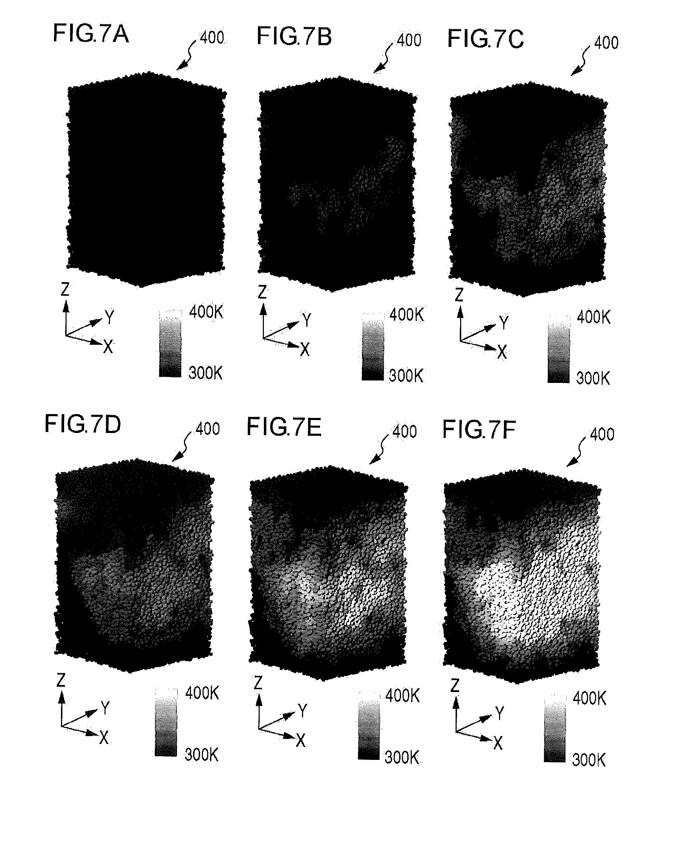

We conducted a calculation to certify the method according to this example. Figs. 7A-7F are schematic diagrams showing results of calculation obtained when the method according to this example is used. A particle system 400 simulates a metal block of 0.6 mm (X direction)×0.6 mm (Y direction)×0.95 mm (Z direction). Figs. 7A-7F show the time-dependent change occurring when a pull force equivalent to 50 GPa is exerted on one upper layer and one lower layer of the particle system 400. Fig. 7A shows the moment when the pull force is exerted on the particle system 400. Figs. 7B, 7C, 7D, 7E, and 7F show the state occurring after the elapse of 25 μs, 50 μs, 75 μs, 100 μs, and 125 μs since the time evolution of the particle system 400 is started. The results show that the temperature of the particle system 400 is increased as a result of the exertion of the pull force and the associated variation in the structure of the particle system 400. This is in agreement with the knowledge that variation in the structure of the particle system 400 generates heat.

The structure and operation of the analyzing device 100 according to the examples are described above. The examples are intended to be illustrative only and it will be obvious to those skilled in the art that various modifications to combinations of constituting elements and processes could be developed and that such modifications are also within the scope of the present invention.

The repeated calculation unit 120 according to the examples are described as calculating both the position and velocity of the particle. However, the description is non-limiting as to the mode of calculation. For example, some numerical analysis methods like the Verlet method directly calculate the position of a particle by referring to the force exerted on the particle and so do not require positively calculating the velocity of the particle. The technical idea according to the examples may also be applied to such methods.

[DESCRIPTION OF THE REFERENCE NUMERALS]

100 analyzing device, 102 input device, 104 display, 108 particle system acquisition unit, 114 particle data storing unit, 118 display control unit, 120 repeated calculation unit

[INDUSTRIAL APPLICABILITY]

According to the examples of the invention, heat conduction phenomenon can be handled accurately in a simulation using a particle system including a plurality of particles.

**Claims**

1. An analyzing device that analyzes a particle system defined in a virtual system by numerically calculating a governing equation that governs motion of particles in the particle system, comprising:

   a temperature calculation unit that calculates a temperature of a particle, which is one of parameters of particles in the particle system;
   a force calculation unit that calculates a force exerted on the particle assumed to be immersed in a heat bath of the temperature calculated by the temperature calculation unit; and
   a state update unit that updates a state of the particle based on the force calculated by the force calculation unit.

2. The analyzing device according to claim 1, wherein
   the temperature calculation unit includes:

   a Voronoi processing unit that creates a Voronoi polyhedron in the virtual space based on a position of a particle; and
   a heat conduction calculation unit that calculates a temperature of the particle based on a heat conduction equation discretized by using the Voronoi polyhedron created by the Voronoi processing unit as a unit.

3. The analyzing device according to claim 2, wherein
   the heat conduction equation includes a term indicating heat generation due to deformation of the particle system.

4. The analyzing device according to one of claims 1 through 3, wherein
   material constants are defined for a particle in the particle system so that the particle simulates a metal particle.

5. A computer program embedded in a non-transitory computer readable recording medium to implement on a computer a function of analyzing a particle system defined in a virtual system by numerically calculating a governing equation that governs motion of particles in the particle system, the program comprising:

   a temperature calculation module that calculates a temperature of a particle, which is one of parameters of particles in the particle system;
   a force calculation module that calculates a force exerted on the particle assumed to be immersed in a heat bath of the temperature calculated by the temperature calculation unit; and
   a state updating module that updates a state of the particle based on the force calculated by the force calculation unit.

FIG.1

102

INPUT DEVICE

ANALYZING DEVICE

114

108 — PARTICLE SYSTEM
ACQUISITION UNIT

134 — TEMPERATURE
ASSOCIATION UNIT

DISPLAY

PARTICLE
DATA STORING
UNIT

104

120 — REPEATED
CALCULATION UNIT

110 — TEMPERATURE
CALCULATION UNIT

112 — VORONOI
DIVISION UNIT

116 — HEAT CONDUCTION
CALCULATION UNIT

122 — FORCE
CALCULATION UNIT

130 — INTER-PARTICLE
ACTION
CALCULATION UNIT

132 — HEAT BATH ACTION
CALCULATION UNIT

124 — PARTICLE STATE
CALCULATION UNIT

126 — STATE
UPDATE UNIT

128 — TERMINATION
CONDITION
DECIDING UNIT

118 — DISPLAY
CONTROL UNIT

100

FIG.2

| PARTICLE ID | POSITION | VELOCITY | TEMPERATURE |
|---|---|---|---|
| 1 | (10,20,30) | (10,10,0) | 125 |
| 2 | (11,22,32) | (2,3,1) | 130 |
| 3 | (31,42,50) | (5,15,2) | 135 |
| | | | |

114

FIG.3

```
                    ┌─────────────┐
                    │    START    │
                    └──────┬──────┘
                           │
          ┌────────────────────────────────┐
          │  DETERMINE INITIAL STATE        │ ～S202
          │    OF PARTICLE SYSTEM           │
          └────────────────┬───────────────┘
                           │←───────────────────────────┐
          ┌────────────────────────────────┐            │
          │      VORONOI ANALYSIS           │ ～S204     │
          └────────────────┬───────────────┘            │
                           │                             │
          ┌────────────────────────────────┐            │
          │   ANALYZE TEMPERATURE           │ ～S206     │
          │     FIELD USING FVM             │            │
          └────────────────┬───────────────┘            │
                           │                             │
          ┌────────────────────────────────┐            │
          │ CALCULATE FORCE EXERTED ON      │ ～S208     │
          │ PARTICLES BASED ON POTENTIAL    │            │
          │     BETWEEN PARTICLES           │            │
          └────────────────┬───────────────┘            │
                           │                             │
          ┌────────────────────────────────┐            │
          │   ADD RANDOM FORCE              │ ～S210     │
          │   AND VISCOUS FORCE             │            │
          └────────────────┬───────────────┘            │
                           │                             │
          ┌────────────────────────────────┐            │
          │ CALCULATE POSITION AND VELOCITY │ ～S212     │
          │  OF PARTICLES BASED ON MOTION   │            │
          │     EQUATION OF PARTICLES       │            │
          └────────────────┬───────────────┘            │
                           │                             │
          ┌────────────────────────────────┐            │
          │   UPDATE POSITION AND           │ ～S214     │
          │   VELOCITY OF PARTICLES         │            │
          └────────────────┬───────────────┘            │
                           │            S216             │
                      ╱────┴────╲                        │
                    ╱             ╲                      │
                  ╱  IS TERMINATION ╲   N                │
                  ╲ CONDITION MET?  ╱──────────────────┘
                    ╲             ╱
                      ╲────┬────╱
                          Y│
                    ┌──────┴──────┐
                    │     END     │
                    └─────────────┘
```

FIG.4

FIG.5

FIG.6

FIG.7A

FIG.7B

FIG.7C

FIG.7D

FIG.7E

FIG.7F

**INTERNATIONAL SEARCH REPORT**

| International application No. |
|---|
| PCT/JP2013/005581 |

A. CLASSIFICATION OF SUBJECT MATTER
*G06F19/00*(2011.01)i, *G01N15/00*(2006.01)i

According to International Patent Classification (IPC) or to both national classification and IPC

B. FIELDS SEARCHED

Minimum documentation searched (classification system followed by classification symbols)
G06F19/00, G01N15/00

Documentation searched other than minimum documentation to the extent that such documents are included in the fields searched
Jitsuyo Shinan Koho        1922-1996   Jitsuyo Shinan Toroku Koho   1996-2013
Kokai Jitsuyo Shinan Koho  1971-2013   Toroku Jitsuyo Shinan Koho   1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2011-175327 A  (Sumitomo Heavy Industries, Ltd.), 08 September 2011 (08.09.2011), paragraphs [0012] to [0083] (Family: none) | 1-5 |
| Y | JP 2010-170309 A  (Sumitomo Heavy Industries, Ltd.), 05 August 2010 (05.08.2010), claim 1 (Family: none) | 1-5 |
| Y | JP 2012-128490 A  (Sumitomo Heavy Industries, Ltd.), 05 July 2012 (05.07.2012), paragraphs [0051] to [0129] (Family: none) | 1-5 |

| ☒  Further documents are listed in the continuation of Box C. | ☐  See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered  to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 December, 2013 (03.12.13) | 17 December, 2013 (17.12.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2013/005581

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2012-150673 A  (Sumitomo Heavy Industries, Ltd.), 09 August 2012 (09.08.2012), claim 1 (Family: none) | 1-5 |
| Y | JP 2010-230331 A  (Netsuren Co., Ltd.), 14 October 2010 (14.10.2010), paragraphs [0019] to [0022] (Family: none) | 3,4 |
| Y | JP 2001-334305 A  (NKK Corp.), 04 December 2001 (04.12.2001), paragraphs [0029] to [0058] (Family: none) | 3,4 |
| Y | JP 61-132205 A  (Kawasaki Steel Corp.), 19 June 1986 (19.06.1986), page 3, lower right column, line 4 to page 4, upper right column, line 5 (Family: none) | 3,4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010170309 A

- JP 2012150673 A

**Non-patent literature cited in the description**

- **JOHN C. TULLY.** Dynamics of gas-surface interaction: 3D generalized Langevin model applied to fcc and bcc surface. *J. Chem. Phys.*, 1975, 73